# FASCICULE DE BREVET EUROPEEN

(11) **EP 3 844 484 B1**
(45) Date de publication et mention de la délivrance du brevet: **20.12.2023**
(21) Numéro de dépôt: 19774138.2
(22) Date de dépôt: 26.08.2019
(51) Int. Cl.: G01N 21/31, G01N 21/17, G01W 1/04, G01N 15/06, G01N 33/00, G01N 15/00

(54) **PROCÉDÉ ET DISPOSITIF DE MESURE DE PARAMÈTRES ATMOSPHÉRIQUES POUR ESTIMER LA QUALITÉ DE L'AIR ET LES VARIABLES CLIMATIQUES**
VERFAHREN UND VORRICHTUNG ZUR MESSUNG VON ATMOSPHÄRISCHEN PARAMETERN ZUR SCHÄTZUNG VON LUFTQUALITÄT UND KLIMAGRÖSSEN
METHOD AND DEVICE FOR MEASURING ATMOSPHERIC PARAMETERS TO ESTIMATE AIR QUALITY AND CLIMATE VARIABLES

(30) Priorité: 27.08.2018 FR 1857683
(43) Date de publication de la demande: 07.07.2021
(73) Titulaire: Centre National d'Etudes Spatiales, 75039 Paris Cedex 01 (FR)
(72) Inventeur: PEQUIGNOT, Eric, 31450 POMPERTUZAT (FR)
(74) Mandataire: Germain Maureau
(86) Numéro de dépôt international: PCT/FR2019/051961
(87) Numéro de publication internationale: WO 2020/043983

(56) Documents cités:
- FR-A1- 2 988 486
- US-A1- 2010 241 361

## Description

La présente invention se rapporte au domaine de la mesure de paramètres atmosphériques, notamment à des fins de surveillance, de prévision et de gestion de la qualité de l'air.

On connaît des techniques permettant de surveiller la qualité de l'air par mesure de concentrations de polluants dans des échantillons d'air prélevés par des stations de mesure. Par exemple, le territoire français est équipé de plusieurs centaines de stations de ce type. De telles stations de mesure constituent toutefois des infrastructures coûteuses et dont la densité est relativement faible sur le territoire.

Les échantillons d'air ainsi prélevés sont généralement utilisés pour estimer la qualité de l'air à l'aide de modèles numériques de chimie transport. Ces modèles numériques nécessitent des temps de calcul importants, conduisant à une limitation de la zone géographique étudiée et/ou du nombre de stations de mesure mis en oeuvre dans la zone d'intérêt.

Ce faisant, pour estimer des niveaux de pollution à de fines échelles spatiales, il est nécessaire de recourir à des techniques de lissage et de propagation des données de mesure.

De telles techniques se traduisent par une sous-estimation des niveaux de pollution et plus généralement par une qualité insatisfaisante des analyses et prévisions auxquelles elles donnent lieu.

FR2988486 A1 divulgue un procédé, et un dispositif de sondage de l'atmosphère terrestre, comprenant un instrument de mesure de rayonnement infrarouge, pour effectuer une étude tridimensionnelle de l'atmosphère d'une planète pour des applications de prévision météorologique dans lesquelles des informations de redondance dans les images spectroscopiques acquises sont générées par l'intersection des deux lignes de visée au point d'intersection, et la partie des informations de redondance causée par les intersections des lignes est utilisée pour augmenter la résolution horizontale de l'image spectroscopique.

Un but de la présente invention est de surmonter les inconvénients précités en proposant un procédé et un dispositif capables d'améliorer la qualité des analyses et prévisions de qualité de l'air, tout en réduisant le coût des infrastructures et de leur mise en oeuvre.

A cet effet, l'invention se rapporte, selon un premier aspect, à un procédé de mesure d'au moins un paramètre atmosphérique, comprenant une étape d'acquisition et une étape de balayage telles que définies dans la revendication 1.

Dans l'étape d'acquisition, on acquiert simultanément une série d'images spectrales de l'atmosphère terrestre avec des systèmes optiques, en particulier des systèmes optiques multi-spectraux travaillant dans un domaine de longueur d'onde allant de l'ultra-violet à l'infrarouge. Par exemple, ces systèmes optiques peuvent être des imageurs et/ou des radiomètres et/ou des spectro-imageurs et/ou des spectro-radiomètres.

Par l'expression « image spectrale », ou « image spectrométrique », on entend une image pouvant typiquement comprendre une matrice de *N* * M pixels, avec *N* ≥ 1 et *M* ≥ 1. Par exemple, dans le cas particulier d'une image acquise à l'aide d'un radiomètre, *N* et *M* peuvent être chacun égaux à 1 de sorte que cette image comprenne un unique pixel.

D'une part, les systèmes optiques mis en oeuvre dans le procédé selon l'invention sont situés dans l'atmosphère terrestre et sont fixes relativement à un référentiel terrestre.

D'autre part, les systèmes optiques mis en oeuvre dans ce procédé sont orientés de sorte que les images spectrales qu'ils acquièrent contiennent des données de mesure dudit au moins un paramètre atmosphérique le long de lignes de visée respectives desdits systèmes optiques.

D'autre part encore, ces systèmes optiques sont agencés de sorte que, lors de l'étape d'acquisition, il existe au moins un point d'intersection entre deux lignes de visée, de sorte que deux images spectrales simultanément acquises suivant ces deux lignes de visée contiennent des données mutuelles de mesure de l'au moins un paramètre atmosphérique, ces données mutuelles de mesure étant représentatives de l'au moins un paramètre atmosphérique en un point de l'atmosphère terrestre correspondant audit point d'intersection.

Selon l'invention, l'orientation des systèmes optiques est modifiée lors de l'étape de balayage, et les étapes d'acquisition et de balayage sont répétées de manière à acquérir une succession de séries d'images spectrales comprenant un ensemble de données mutuelles de mesure représentatif de l'au moins un paramètre atmosphérique en un ensemble de points de l'atmosphère terrestre.

Un tel procédé permet de réaliser des mesures de paramètres atmosphériques à l'aide d'infrastructures peu coûteuses et améliorant la qualité des analyses et prévisions de la qualité de l'air, en particulier à l'échelle d'une zone géographique dont l'étendue est relativement réduite telle qu'une agglomération urbaine.

A titre d'exemples non limitatifs, l'au moins un paramètre atmosphérique peut être choisi parmi la température atmosphérique et/ou parmi au moins un gaz atmosphérique ayant une signature dans l'ultra-violet et/ou le visible et/ou l'infrarouge tel que O₃, NO₂, SO₂, CO, PM1, PM2.5, PM10, H₂O, CO₂, CH₄, N₂O, NH₃.

En effet, les systèmes optiques de l'invention permettent de mesurer des paramètres scientifiques d'intérêt faisant l'objet d'une législation (p. ex. O₃, NO₂, SO₂, CO ; particules fines PM1, PM2.5, PM10) et/ou faisant l'objet de pics de pollution récurrents (p. ex. O₃, NO₂, PM2.5, PM10), ainsi que des produits complémentaires tels que des champs météorologiques (p. ex. T, H₂O), des gaz à effet de serre (p. ex. CO₂, CH₄, N₂O), des marqueurs de feux par exemple de forêt (p. ex. SO₂, CO) ou encore des polluants agricoles (p. ex. NH₃).

Dans un mode de réalisation, lors de l'étape de balayage, l'orientation des systèmes optiques peut être modifiée de sorte que, entre l'étape d'acquisition précédant cette étape de balayage et l'étape d'acquisition succédant à cette étape de balayage, ledit au moins un point d'intersection passe d'une première coordonnée géographique à une deuxième coordonnée géographique, cette deuxième coordonnée géographique étant différente de ladite première coordonnée géographique en longitude et/ou en latitude et/ou en élévation.

Selon un deuxième aspect, l'invention concerne aussi un procédé d'étude d'au moins un paramètre atmosphérique mettant en oeuvre le procédé de mesure selon la revendication 3.

De préférence, ce procédé d'étude d'au moins un paramètre atmosphérique peut comprendre en outre une étape d'analyse des images spectrales acquises. Une telle étape d'analyse comprend préférentiellement une étape d'inversion des images spectrales exploitant au moins une partie de l'ensemble de données mutuelles de mesure contenues dans ces images spectrales.

Selon un troisième aspect, l'invention concerne aussi un dispositif de mesure d'au moins un paramètre atmosphérique selon la revendication 4.

Comme indiqué ci-dessus, les systèmes optiques peuvent être des imageurs et/ou des radiomètres et/ou des spectro-imageurs et/ou des spectro-radiomètres. Autrement dit, les systèmes optiques peuvent être des caméras infrarouges.

La distance entre deux systèmes optiques adjacents peut typiquement être comprise entre 10 m et 20 km, de préférence entre 2 km et 5 km, plus préférentiellement égale à 3 km.

Selon une première variante, les systèmes optiques peuvent être fixés sur des supports respectifs solidaires du sol, tels que des pylônes, des bâtiments, des châteaux d'eau ou encore des ballons captifs.

On peut ainsi utiliser des infrastructures déjà existantes.

Selon une deuxième variante, les systèmes optiques peuvent être embarqués sur un ou plusieurs aéronefs stationnaires, tels que des drones ou des ballons atmosphériques.

Selon un quatrième aspect, l'invention concerne aussi l' utilisation d'un procédé ou d'un dispositif selon la revendication 9 pour estimer la qualité de l'air et/ou des paramètres météorologiques et/ou climatiques d'une zone géographique dont l'étendue est de préférence comprise entre 100 m et 100 km. De manière non limitative, cette zone géographique peut être une agglomération urbaine, un site industriel, une forêt ou encore un site agricole.

La présente invention permet ainsi de constituer un réseau tomographique de télédétection au sol capable de mesurer la qualité de l'air urbaine à l'échelle résidentielle. Cette solution permet, par exemple, d'informer des usagers ou habitants de leur exposition individuelle aux polluants de l'air grâce à une mesure fiable et en continue à l'échelle locale.

D'autres caractéristiques et avantages de l'invention apparaîtront à la lecture de la description nullement limitative qui suit et des figures annexées, dans lesquelles :
- La figure 1 représente de manière schématique un réseau de systèmes optiques selon l'invention dans une première configuration ;
- La figure 2 montre le réseau de systèmes optiques de la figure 1 dans une deuxième configuration.

Les éléments identiques sont repérés par des signes de référence identiques sur l'ensemble des figures.

L'invention concerne un dispositif et un procédé de mesure d'au moins un paramètre atmosphérique mettant en oeuvre une étape d'acquisition simultanée d'une série d'images spectrales de l'atmosphère terrestre à l'aide de systèmes optiques.

Le ou les paramètres atmosphériques peuvent par exemple consister en toute combinaison de gaz atmosphériques ayant une signature dans l'ultra-violet et/ou l'infrarouge tels que O₃, NO₂, SO₂, CO, PM1, PM2.5, PM10, H₂O, CO₂, CH₄, N₂O, NH₃. Un autre exemple de paramètre atmosphérique est la température atmosphérique.

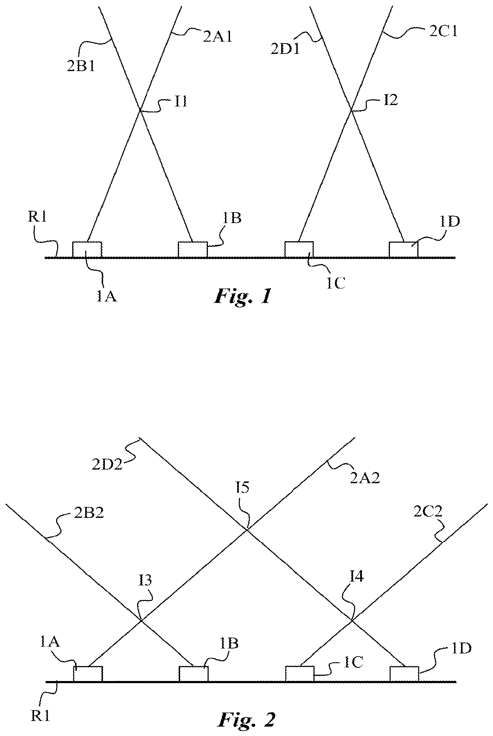

Une étape d'acquisition est schématiquement illustrée à la figure 1.

La figure 1 montre un réseau de quatre systèmes optiques 1A-1D. Ces systèmes optiques 1A-1D sont typiquement des caméras telles que des imageurs, des radiomètres, des spectro-imageurs, ou des spectro-radiomètres, capables d'acquérir des images spectrales dans un domaine de longueur d'onde allant de l'ultra-violet à l'infrarouge.

Les systèmes optiques 1A-1D sont situés dans l'atmosphère terrestre et sont fixes relativement à un référentiel terrestre R1. Par l'expression « systèmes optiques fixes », on entend des systèmes optiques ayant une position relative par rapport audit référentiel terrestre R1 qui est invariable lors de la mise en oeuvre du procédé de l'invention, indépendamment de l'orientation de ces systèmes optiques qui peut être modifiée lors de la mise en oeuvre du procédé de l'invention.

Pour ce faire, les systèmes optiques 1A-1D peuvent être montés sur des supports respectifs solidaires du sol, tels que des pylônes, des bâtiments, des châteaux d'eau ou encore des ballons captifs. De préférence, ces supports disposent d'une alimentation électrique et/ou internet afin d'alimenter les systèmes optiques. Le dispositif peut aussi comprendre des moyens d'alimentation du type batterie solaire, et/ou des moyens de connexion wifi par exemple 3G ou 4G, et/ou des moyens de connexion à distance de tout autre type.

Alternativement, les systèmes optiques 1A-1D peuvent être embarqués sur un ou plusieurs aéronefs stationnaires, tels que des drones ou des ballons atmosphériques.

A titre d'exemple non limitatif, la distance entre deux systèmes optiques adjacents peut être d'environ 3 km. Plus généralement, en fonction de la zone géographique à étudier, cette distance peut être comprise entre 10 m et 20 km. Bien entendu, la distance entre chaque paire de systèmes optiques adjacents peut être identique ou différente. Dans l'exemple de la figure 1, la distance entre les systèmes optiques 1A et 1B est identique à la distance entre les systèmes optiques 1B et 1C et à la distance entre les systèmes optiques 1C et 1D.

En référence à la figure 1, les systèmes optiques 1A-1D sont orientés de sorte que les images spectrales qu'ils acquièrent contiennent des données de mesure d'au moins un paramètre atmosphérique le long de lignes de visée 2A1-2D1 respectives desdits systèmes optiques 1A-1D.

Autrement dit, à chacun des systèmes optiques 1A-1D correspond une ligne de visée 2A1-2D1 respective le long de laquelle chacun de ces systèmes optiques 1A-1D acquiert une image spectrale ou une série d'images spectrales. En particulier, le système optique 1A acquiert une image spectrale ou une série d'images spectrales le long de la ligne de visée 2A1, le système optique 1B acquiert une image spectrale ou une série d'images spectrales le long de la ligne de visée 2B1, etc. (voir figure 1).

Lors de l'étape d'acquisition dans la configuration illustrée à la figure 1, les systèmes optiques 1A-1D sont agencés de sorte qu'il existe au moins un point d'intersection entre deux lignes de visée. Dans cet exemple, il existe un point d'intersection I1 entre les lignes de visée 2A1 et 2B1, et un point d'intersection I2 entre les lignes de visée 2C1 et 2D1.

Un tel agencement permet l'acquisition simultanée de deux images spectrales par les systèmes optiques 1A et 1B, suivant les lignes de visée 2A1 et 2B1. Ces deux images spectrales contiennent des données mutuelles de mesure de l'au moins un paramètre atmosphérique. Ces données mutuelles de mesure sont représentatives de l'au moins un paramètre atmosphérique en un point de l'atmosphère terrestre correspondant dans ce cas au point d'intersection I1.

De même, un tel agencement permet l'acquisition simultanée de deux images spectrales par les systèmes optiques 1C et 1D, suivant les lignes de visée 2C1 et 2D1. Ces deux images spectrales contiennent des données mutuelles de mesure de l'au moins un paramètre atmosphérique. Ces données mutuelles de mesure sont représentatives de l'au moins un paramètre atmosphérique en un point de l'atmosphère terrestre correspondant dans ce cas au point d'intersection I2.

Ainsi, lors de l'étape d'acquisition dans la configuration illustrée à la figure 1, une première série comprenant deux images spectrales est acquise, cette série d'images spectrales comprenant un ensemble de données mutuelles de mesure représentatif de l'au moins un paramètre atmosphérique en un ensemble de deux points de l'atmosphère terrestre correspondants aux points d'intersection I1 et I2.

L'invention permet d'acquérir une succession de séries d'images spectrales grâce à la mise en oeuvre d'une étape de balayage, selon un principe tomographique.

L'étape de balayage consiste à modifier l'orientation des systèmes optiques 1A-1D tel qu'illustré par la modification de l'orientation des lignes de visée associées à ces systèmes optiques entre configuration de la figure 1 et la configuration de la figure 2.

Selon l'invention, on répète successivement de telles étapes d'acquisition et de balayage de manière à acquérir une succession de séries d'images spectrales comprenant un ensemble de données mutuelles de mesure représentatif de l'au moins un paramètre atmosphérique en un ensemble de points de l'atmosphère terrestre.

Dans l'exemple des figures 1 et 2, après acquisition des deux images spectrales dans la configuration illustrée à la figure 1 puis modification de l'orientation des systèmes optiques 1A-1D pour parvenir à la configuration illustrée à la figure 2, on réalise une étape d'acquisition dans la configuration de la figure 2.

Plus précisément, dans la configuration de la figure 2, les systèmes optiques 1A-1D sont agencés de sorte qu'il existe un point d'intersection I3 entre les lignes de visée 2A2 et 2B2, un point d'intersection I4 entre les lignes de visée 2C2 et 2D2, et un point d'intersection I5 entre les lignes de visée 2A2 et 2D2. Les lignes de visées 2A2, 2B2, 2C2 et 2D2 correspondent respectivement aux systèmes optiques 1A, 1B, 1C et 1D dans la configuration de la figure 2.

Dans cet agencement, on peut ainsi acquérir simultanément deux images spectrales suivant les lignes de visée 2A2 et 2B2 contenant des données mutuelles de mesure de l'au moins un paramètre atmosphérique. Ces données mutuelles de mesure sont représentatives de l'au moins un paramètre atmosphérique en un point de l'atmosphère terrestre correspondant au point d'intersection I3.

L'agencement illustré à la figure 2 permet aussi l'acquisition simultanée de deux images spectrales suivant les lignes de visée 2C2 et 2D2 contenant des données mutuelles de mesure de l'au moins un paramètre atmosphérique. Ces données mutuelles de mesure sont représentatives de l'au moins un paramètre atmosphérique en un point de l'atmosphère terrestre correspondant au point d'intersection I4.

De plus, dans cette configuration, les deux images spectrales acquises par les systèmes optiques 1A et 1D, suivant les lignes de visée 2A2 et 2D2, contiennent en outre des données mutuelles de mesure représentatives de l'au moins un paramètre atmosphérique en un point de l'atmosphère terrestre correspondant au point d'intersection I5.

Par conséquent, l'étape d'acquisition réalisée dans la configuration illustrée à la figure 2 permet d'acquérir une deuxième série de deux images spectrales comprenant un ensemble de données mutuelles de mesure représentatif de l'au moins un paramètre atmosphérique en un ensemble de trois points de l'atmosphère terrestre correspondants aux points d'intersections I3, I4 et I5.

La répétition successive de telles étapes d'acquisition et de balayage peut être réalisée de sorte que l'ensemble de données mutuelles de mesure soit représentatif de l'au moins un paramètre atmosphérique en un ensemble de points de l'atmosphère terrestre situés dans un plan sensiblement horizontal et/ou dans un plan sensiblement vertical et/ou dans un volume de l'atmosphère terrestre.

Pour ce faire, l'orientation des systèmes optiques 1A-1D lors d'une étape de balayage peut être modifiée de sorte que, d'une étape d'acquisition antérieure à cette étape de balayage à une étape d'acquisition postérieure à cette étape de balayage, un ou plusieurs points d'intersection passent chacun d'une première coordonnée géographique à une deuxième coordonnée géographique, cette deuxième coordonnée géographique étant différente de ladite première coordonnée géographique en longitude et/ou en latitude et/ou en élévation.

La densité des lignes de visée et des points d'intersection, c'est-à-dire le nombre de répétition des étapes d'acquisition et de balayage ainsi que le pas ou incrément de l'angle d'orientation des systèmes optiques, peuvent être adaptés en fonction de la topologie de la zone d'intérêt, des résolutions horizontale et verticale souhaitées, de la densité des systèmes optiques et/ou de l'horizon optique de chaque bande ou canal spectral de chaque système optique.

Les systèmes optiques sont de préférence disposés en hauteur, c'est-à-dire dans une configuration maximisant l'horizon géométrique.

Dans un exemple de réalisation, les systèmes optiques 1A-1D sont montés sur des tourelles robotisées (non représentées) permettant d'orienter ces systèmes optiques suivant un angle compris entre -180° et +180° dans une direction longitudinale et suivant un angle compris entre 0° et +90° dans une direction latitudinale. L'angle latitudinal est compté par rapport à l'horizontale au sol positivement vers le zénith. Un tel mode de réalisation permet de scanner un demi-hémisphère supérieur.

Dans un autre mode de réalisation, l'angle latitudinal peut être compris entre - 90° et +90° de manière à scanner aussi un demi-hémisphère inférieur, et d'évaluer ainsi l'apport éventuel de la mesure de la réflectance de surface ainsi que son incidence dans le transfert radiatif (modélisation directe) et dans l'inversion (voir ci-dessous).

Tout type de scannage peut être envisagé et les exemples de réalisation qui précèdent ne sont aucunement limitatifs.

La présente invention permet d'étudier des paramètres atmosphériques par analyse des images spectrales acquises selon les principes décrits ci-dessus.

A cet effet, on met de préférence en oeuvre une étape d'analyse comprenant une étape d'inversion des images spectrales, en exploitant au moins une partie de l'ensemble de données mutuelles de mesure contenues dans ces images spectrales.

L'analyse peut être réalisée à l'aide d'un logiciel d'inversion multi-lignes de visée bayésien.

Afin de tenir compte de l'évolution temporelle de l'au moins un paramètre atmosphérique, l'analyse peut être réalisée de manière séquentielle, en travaillant en anomalie entre le temps t et le temps t - 1 h. Par exemple, les apriori peuvent être fournis par les champs calculés à t - 1 h pour les gaz et à l'estimation faite à t pour des produits complémentaires du type champs météorologiques tels que T et H₂O. L'apriori initial peut être issu d'une analyse ou prévision réalisée par des centres de météorologie sur la zone géographique étudiée.

Un traitement temporel séquentiel permet de s'affranchir d'une partie des évolutions des variables géophysiques (variables atmosphériques ou caractéristiques des surfaces réfléchissantes) et donc de conserver un modèle direct linéaire. Cela permet aussi d'accélérer considérablement les temps de calcul.

Bien entendu, l'invention n'est pas limitée aux exemples particuliers qui viennent d'être décrits et l'homme du métier pourra réaliser toute adaptation ou mettre en oeuvre des étapes ou caractéristiques supplémentaires dans le cadre des revendications annexées.

Ainsi, on pourra mettre en oeuvre une étape de calibration des caméras, par exemple à l'aide d'émetteurs de lumière dont le spectre est bien caractérisé (calibration radiométrique) ou encore en utilisant des cibles noires et blanches dotées de motifs géométriques prédéfinis (calibration géométrique).

Entre autres avantages, cette invention permet :
- d'estimer la qualité de l'air avec une résolution de mesure spatio-temporelle résidentielle inférieure à 250 m/h,
- de produire des résultats d'étude en temps réel par une mesure directe plutôt que par un modèle numérique, cela avec une précision de l'ordre de 15-25%,
- de couvrir complètement une agglomération urbaine avec un réseau de stations de mesure quatre-cent fois plus dense que les réseaux existants,
- de réaliser une caractérisation tridimensionnelle de l'atmosphère,
- d'accéder à des produits complémentaires tels que des champs météorologiques (p. ex. T, H₂O), des gaz à effet de serre (p. ex. CO₂, CH₄, N₂O), des marqueurs de feux par exemple de forêt (p. ex. SO₂, CO) ou encore des polluants agricoles (p. ex. NH₃).

Les champs d'application de cette invention sont nombreux et comprennent notamment la surveillance locale et en continue de la qualité de l'air urbaine, le suivi de sites industriels par exemple par ciblage de gaz à effet de serre, la sécurité civile par exemple par détection de feux de forêt, l'agriculture, la thermographie infrarouge de bâtiments, la valorisation territoriale, la santé par exemple par étude de corrélation entre pollution et prévalence de maladies...

## Revendications

1. Procédé de mesure d'au moins un paramètre atmosphérique choisi parmi la température atmosphérique et/ou au moins un gaz atmosphérique ayant une signature dans l'ultra-violet et/ou le visible et/ou l'infrarouge tel que O₃, NO₂, SO₂, CO, PM1, PM2.5, PM10, H₂O, CO₂, CH₄, N₂O ou NH₃, le procédé comprenant:
- une étape d'acquisition dans laquelle on acquiert simultanément une série d'images spectrales de l'atmosphère terrestre avec des systèmes optiques (1A-1D) multi-spectraux travaillant dans un domaine de longueur d'onde allant de l'ultra-violet à l'infrarouge :
∘ situés dans l'atmosphère terrestre,
∘ fixes relativement à un référentiel terrestre (R1),
∘ orientés de sorte que les images spectrales qu'ils acquièrent contiennent des données de mesure dudit au moins un paramètre atmosphérique le long de lignes de visée (2A1-2D1) respectives desdits systèmes optiques (1A-1D),
∘ agencés de sorte que, lors de cette étape d'acquisition, il existe au moins un point d'intersection (I1 ; I2) entre deux lignes de visée (2A1, 2B1 ; 2C1, 2D1), de sorte que deux images spectrales simultanément acquises suivant ces deux lignes de visée (2A1, 2B1 ; 2C1, 2D1) contiennent des données mutuelles de mesure de l'au moins un paramètre atmosphérique, ces données mutuelles de mesure étant représentatives de l'au moins un paramètre atmosphérique en un point de l'atmosphère terrestre correspondant audit point d'intersection (I1 ; I2),
- une étape de balayage dans laquelle l'orientation des systèmes optiques (1A-1D) est modifiée,
et en ce que l'étape d'acquisition et l'étape de balayage sont répétées de manière à acquérir une succession de séries d'images spectrales comprenant un ensemble de données mutuelles de mesure représentatif de l'au moins un paramètre atmosphérique en un ensemble de points (I1-I5) de l'atmosphère terrestre.

2. Procédé selon la revendication 1, dans lequel, lors de l'étape de balayage, l'orientation des systèmes optiques (1A-1D) est modifiée de sorte que, entre l'étape d'acquisition précédant cette étape de balayage et l'étape d'acquisition succédant à cette étape de balayage, ledit au moins un point d'intersection passe d'une première coordonnée géographique (I1) à une deuxième coordonnée géographique (I3), cette deuxième coordonnée géographique (I3) étant différente de cette première coordonnée géographique (I1) en longitude et/ou en latitude et/ou en élévation.

3. Procédé d'étude d'au moins un paramètre atmosphérique, **caractérisé en ce qu'**il met en oeuvre le procédé de mesure selon l'une quelconque des revendications précédentes, et **en ce qu'**il comprend en outre une étape d'analyse des images spectrales acquises, cette étape d'analyse comprenant une étape d'inversion de ces images spectrales exploitant au moins une partie de l'ensemble de données mutuelles de mesure contenues dans ces images spectrales.

4. Dispositif de mesure d'au moins un paramètre atmosphérique comprenant un réseau de systèmes optiques (1A-1D) agencés pour mettre en oeuvre un procédé selon l'une quelconque des revendications 1 à 2, les systèmes optiques (1A-1D) multi-spectraux adaptés pour travailler dans un domaine de longueur d'onde allant de l'ultra-violet à l'infrarouge, les systèmes optiques (1A-1D) étant:
∘ situés dans l'atmosphère terrestre,
∘ fixes relativement à un référentiel terrestre (R1),
∘ orientés de sorte que les images spectrales qu'ils acquièrent contiennent des données de mesure dudit au moins un paramètre atmosphérique le long de lignes de visée (2A1-2D1) respectives desdits systèmes optiques (1A-1D).

5. Dispositif selon la revendication 4, dans lequel la distance entre deux systèmes optiques (1A-1D) adjacents est comprise entre 10 m et 20 km, de préférence entre 2 km et 5 km, plus préférentiellement égale à 3 km.

6. Dispositif selon la revendication 4 ou 5, dans lequel les systèmes optiques (1A-1D) sont fixés sur des supports respectifs solidaires du sol, tels que des pylônes, des bâtiments, des châteaux d'eau ou encore des ballons captifs.

7. Dispositif selon la revendication 4 ou 5, dans lequel les systèmes optiques (1A-1D) sont embarqués sur un ou plusieurs aéronefs stationnaires, tels que des drones ou des ballons atmosphériques.

8. Dispositif selon l'une quelconque des revendications 4 à 7, dans lequel les systèmes optiques (1A-1D) sont des imageurs et/ou des radiomètres et/ou des spectro-imageurs et/ou des spectro-radiomètres.

9. Utilisation d'un procédé selon l'une quelconque des revendications 1 à 3 ou d'un dispositif selon l'une quelconque des revendications 4 à 8 pour estimer la qualité de l'air et/ou des paramètres météorologiques et/ou climatiques d'une zone géographique dont l'étendue est de préférence comprise entre 100 m et 100 km telle qu'une agglomération urbaine, un site industriel, une forêt ou un site agricole.

## Patentansprüche

1. Verfahren zum Messen mindestens eines atmosphärischen Parameters, der aus der atmosphärischen Temperatur und/oder mindestens einem atmosphärischen Gas ausgewählt ist, das eine Signatur in Ultraviolett und/oder im sichtbaren Bereich und/oder in Infrarot aufweist, wie O₃, NO₂, SO₂, CO, PM1, PM2.5, PM10, H₂O, CO₂, CH₄, N₂O oder NH₃, wobei das Verfahren umfasst:
- einen Erfassungsschritt, bei dem gleichzeitig eine Reihe von spektralen Bildern der Erdatmosphäre mit multispektralen optischen Systemen (1A-1D) erfasst werden, die in einer Wellenlängendomäne arbeiten, die von Ultraviolett bis Infrarot reicht:
° in der Erdatmosphäre gelegen,
° feststehend in Bezug auf ein terrestrisches Bezugssystem (R1),
° derart ausgerichtet, dass die spektralen Bilder, die sie erfassen Messdaten des mindestens einen atmosphärischen Parameters entlang der jeweiligen Sichtlinien (2A1-2D1) der optischen Systeme (1A-1D) enthalten,
° derart angeordnet, dass bei diesem Erfassungsschritt mindestens ein Schnittpunkt (I1; I2) zwischen zwei Sichtlinien (2A1, 2B1; 2C1, 2D1) vorhanden ist, sodass zwei gleichzeitig entlang der beiden Sichtlinien (2A1, 2B1; 2C1, 2D1) erfasste spektrale Bilder wechselseitige Messdaten des mindestens einen atmosphärischen Parameters enthalten, wobei diese wechselseitigen Messdaten repräsentativ für den mindestens einen atmosphärischen Parameter an einem Punkt der Erdatmosphäre sind, der dem Schnittpunkt (11; I2) entspricht,
- einen Abtastschritt, bei dem die Ausrichtung der optischen Systeme (1A-1D) verändert wird,
und dadurch, dass der Erfassungsschritt und der Abtastschritt derart wiederholt werden, um eine Aufeinanderfolge von Reihen spektraler Bilder zu erfassen, die eine Einheit an wechselseitigen Messdaten umfassen, die repräsentativ für den mindestens einen atmosphärischen Parameter in einer Einheit von Punkten (11-15) der Erdatmosphäre ist.

2. Verfahren nach Anspruch 1, wobei die Ausrichtung der optischen Systeme (1A-1D) beim Abtastschritt derart verändert wird, dass zwischen dem Erfassungsschritt, der diesem Abtastschritt vorangeht, und dem Erfassungsschritt, der diesem Abtastschritt nachfolgt, der mindestens eine Schnittpunkt von einer ersten geografischen Koordinate (11) auf eine zweite geografische Koordinate (I3) übergeht, wobei sich diese zweite geografische Koordinate (I3) von dieser ersten geografischen Koordinate (11) in der Länge und/oder in der Breite und/oder in der Höhe unterscheidet.

3. Verfahren zur Untersuchung mindestens eines atmosphärischen Parameters, **dadurch gekennzeichnet, dass** es das Verfahren zum Messen nach einem der vorstehenden Ansprüche umsetzt, und dadurch, dass es weiter einen Schritt zur Analyse der erfassten spektralen Bilder umfasst, wobei dieser Analyseschritt einen Umkehrschritt dieser spektralen Bilder umfasst, der mindestens einen Teil der Einheit von wechselseitigen Messdaten auswertet, die in diesen spektralen Bildern enthalten sind.

4. Vorrichtung zum Messen mindestens eines atmosphärischen Parameters, umfassend ein Netzwerk an optischen Systemen (1A-1D), die angeordnet sind, um ein Verfahren nach einem der Ansprüche 1 bis 2 umzusetzen, wobei die multispektralen optischen Systeme (1A-1D) angepasst sind, um in einer Wellenlängendomäne zu arbeiten, die von Ultraviolett bis Infrarot reicht, wobei die optischen Systeme (1A-1D) folgendermaßen sind:
° in der Erdatmosphäre gelegen,
° feststehend in Bezug auf ein terrestrisches Bezugssystem (R1),
° derart ausgerichtet, dass die spektralen Bilder, die sie erfassen Messdaten des mindestens einen atmosphärischen Parameters entlang der jeweiligen Sichtlinien (2A1-2D1) der optischen Systeme (1A-1D) enthalten.

5. Vorrichtung nach Anspruch 4, wobei die Distanz zwischen zwei benachbarten optischen Systemen (1A-1D) zwischen 10m und 20km, vorzugsweise zwischen 2km und 5km liegt, und besonders bevorzugt gleich 3km ist.

6. Vorrichtung nach Anspruch 4 oder 5, wobei die optischen Systeme (1A-1D) an jeweiligen Trägern, die fest mit dem Boden verbunden sind, wie Pylonen, Gebäuden, Wassertürmen, oder aber Fesselballonen, befestigt sind.

7. Vorrichtung nach Anspruch 4 oder 5, wobei die optischen Systeme (1A-1D) in einem oder mehreren stationären Luftfahrzeugen, wie Drohnen oder Atmosphären-Ballonen verbaut sind.

8. Vorrichtung nach einem der Ansprüche 4 bis 7, wobei die optischen Systeme (1A-1D) Bildgebungssysteme und/oder Radiometer und/oder spektrale Bildgebungssysteme und/oder spektrale Radiometer sind.

9. Anwendung eines Verfahrens nach einem der Ansprüche 1 bis 3, oder einer Vorrichtung nach einem der Ansprüche 4 bis 8, zum Schätzen der Luftqualität und/oder meteorologischer und/oder klimatischer Parameter einer geografischen Zone deren Ausdehnung vorzugsweise zwischen 100m und 100km liegt, wie eines städtischen Ballungsgebiets, eines Industriestandortes, eines Waldes oder eines landwirtschaftlichen Areals.

## Claims

1. A method for measuring at least one atmospheric parameter chosen from atmospheric temperature and/or at least one atmospheric gas having a signature in the ultraviolet and/or the visible and/or the infrared such as O₃, NO₂, SO₂, CO, PM1, PM2.5, PM10, H₂O, CO₂, CH₄, N₂O or NH₃, the method comprising:
- an acquisition step in which a series of spectral images of the terrestrial atmosphere are simultaneously acquired with multi-spectral optical systems (1A-1D) working in a wavelength domain ranging from ultraviolet to infrared:
o located in the terrestrial atmosphere,
o fastened relative to a terrestrial reference frame (R1),
o oriented so that the spectral images that they acquire contain measurement data of said at least one atmospheric parameter along the respective lines of sight (2A1-2D1) of said optical systems (1A-1D),
o arranged so that, during this acquisition step, there is at least one intersection point (11; I2) between two lines of sight (2A1, 2B1; 2C1, 2D1), so that two spectral images simultaneously acquired along these two lines of sight (2A1, 2B1; 2C1, 2D1) contain mutual measurement data of the at least one atmospheric parameter, these mutual measurement data being representative of the at least one atmospheric parameter at a point of the terrestrial atmosphere corresponding to said intersection point (11; 12),
- a scanning step in which the orientation of the optical systems (1A-1D) is modified,
and in that the acquisition step and the scanning step are repeated so as to acquire a succession of series of spectral images comprising a set of mutual measurement data representative of the at least one atmospheric parameter in a set of points (11-15) of the terrestrial atmosphere.

2. The method according to claim 1, wherein, during the scanning step, the orientation of the optical systems (1A-1D) is modified so that, between the acquisition step preceding this scanning step and the acquisition step succeeding this scanning step, said at least one intersection point passes from a first geographic coordinate (11) to a second geographic coordinate (I3), this second geographic coordinate (13) being different from this first geographic coordinate (11) in longitude and/or latitude and/or elevation.

3. A method for studying at least one atmospheric parameter, **characterized in that** it implements the measurement method according to any one of the preceding claims, and **in that** it further comprises a step of analyzing acquired spectral images, this analysis step comprising a step of inverting these spectral images exploiting at least part of the set of mutual measurement data contained in these spectral images.

4. A device for measuring at least one atmospheric parameter comprising a network of optical systems (1A-1D) arranged to implement a method according to any one of claims 1 and 2, the multi-spectral optical systems (1A-1D) adapted to work in a wavelength domain ranging from ultraviolet to infrared, the optical systems (1A-1D) being:
o located in the terrestrial atmosphere,
o fastened relative to a terrestrial reference frame (R1),
o oriented so that the spectral images that they acquire contain measurement data of said at least one atmospheric parameter along the respective lines of sight (2A1-2D1) of said optical systems (1A-1D).

5. The device according to claim 4, wherein the distance between two adjacent optical systems (1A-1D) is comprised between 10 m and 20 km, preferably between 2 km and 5 km, more preferably equal to 3 km.

6. The device according to claim 4 or 5, wherein the optical systems (1A-1D) are fastened on respective supports secured to the ground, such as pylons, buildings, water towers or even tethered balloons.

7. The device according to claim 4 or 5, wherein the optical systems (1A-1D) are on board one or several stationary aircraft, such as drones or atmospheric balloons.

8. The device according to any one of claims 4 to 7, wherein the optical systems (1A-1D) are imagers and/or radiometers and/or spectro-imagers and/or spectro-radiometers.

9. A use of a method according to any one of claims 1 to 3 or of a device according to any one of claims 4 to 8 to estimate the air quality and/or meteorological and/or climatic parameters of a geographical area whose extent is preferably comprised between 100 m and 100 km such as an urban area, an industrial site, a forest or an agricultural site.
